# EUROPEAN PATENT APPLICATION

(11) **EP 4 438 046 A1**
(43) Date of publication of application: **02.10.2024**
(21) Application number: 22897959.7
(22) Date of filing: 28.11.2022
(51) Int. Cl.: A61K 31/5517, A61P 35/00

(54) **NOVEL APPLICATION OF KINASE INHIBITOR**

(30) Priority: 27.11.2021 CN 202111426502
(71) Applicant: Transthera Sciences (Nanjing), Inc., Jiangbei New Area Nanjing Jiangsu 210032 (CN)
(72) Inventor: PENG, Peng, Nanjing, Jiangsu 210032 (CN); SUN, Caixia, Nanjing, Jiangsu 210032 (CN)
(74) Representative: Winter, Brandl - Partnerschaft mbB
(86) International application number: PCT/CN2022/134614
(87) International publication number: WO 2023/093878

(57) **Abstract**

The present invention belongs to the technical field of medicines, and relates to novel use of a kinase inhibitor. Disclosed is use of a compound of general formula (I) or a pharmaceutically acceptable salt, stereoisomer or crystal form thereof in the manufacture of a medicament for treating and/or preventing prostate cancer, wherein variables in the general formula are as defined in the specification. Researches show that the compound of general formula (I) or the pharmaceutically acceptable salt, stereoisomer or crystal form thereof has a therapeutic effect on prostate cancer, and further has a significant therapeutic effect on castration resistant prostate cancer and castration sensitive prostate cancer as well as metastatic castration resistant prostate cancer.

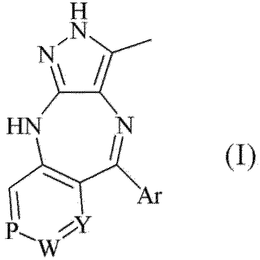

## Description

### TECHNICAL FIELD

The present invention belongs to the technical field of medicines, and particularly relates to novel use of a kinase inhibitor.

### BACKGROUND

Prostate cancer (PCa) is one of the most common malignancies in men. Globally, PCa ranks second in incidence among male tumors, and ranks first in incidence among male malignancies in the United States, with a mortality rate second only to lung cancer.

Based on the staging and risk level of prostate cancer, treatment regimens vary accordingly. For patients with early-stage, low-risk prostate cancer, active surveillance is mainly adopted. Due to reasons such as the lack of obvious symptoms or limitations in detection means for early-stage prostate cancer, most prostate cancers are diagnosed at an advanced stage, with half or more having distant metastases. The 5-year relative survival rate for those with distant metastases is only 30%. For patients with advanced, metastatic prostate cancer, the main treatment means is medical or surgical castration, namely androgen deprivation therapy (ADT). Because the growth of both normal prostate cells and prostate cancer cells is androgen-dependent, the concentration of androgens in a patient's body can be minimized by surgical or medical castration, thereby inhibiting the androgen signaling pathway and ultimately inhibiting the growth of tumor cells. Therapeutic medicaments currently used for ADT include gonadotropin-releasing hormone agonists (GnRH agonists), gonadotropin-releasing hormone antagonists (GnRH antagonists), androgen secretion inhibitors, and the like. Most patients are initially sensitive to ADT, meaning they have castration sensitive prostate cancer (CSPC). However, due to reasons such as genetics or medicament induction, almost all patients will progress to castration resistant prostate cancer (CRPC) after 2 years, developing drug resistance. Once the cancer progresses to CRPC, the median survival time for the patients is only 9-12 months.

CRPC mainly has two independent stages: metastatic castration resistant prostate cancer (mCRPC) with distant metastasis and non-metastatic castration resistant prostate cancer (nmCRPC) without distant metastasis. When patients with nmCRPC experience distant metastases or disease progression after failure of novel endocrine therapies (such as androgen receptor inhibitors), they transition to the mCRPC stage, so mCRPC can be considered the final stage for patients with PCa. The main therapies currently used for mCRPC include chemotherapy (docetaxel), novel endocrine therapies (abiraterone acetate, enzalutamide, etc.), immunotherapy (Sipuleucel-T), and radiotherapy for bone metastases (radium-223). Docetaxel is the first chemotherapeutic medicament approved by the FDA for the treatment of mCRPC, capable of effectively extending the overall survival (OS) of patients by about 19.2 months. However, this medicament causes a series of side effects, such as nausea, vomiting, neurological disorders, anemia, etc. 17α-hydroxylase/C17,20-lyase (CPY17 enzyme) plays a key role in the synthesis of androgens. Abiraterone acetate is a prodrug that is converted into abiraterone *in vivo,* and abiraterone is an androgen biosynthesis inhibitor that can inhibit the CYP17 enzyme. This drug was approved by the FDA in 2011 for use in patients with CRPC. Enzalutamide is a second-generation AR (androgen receptor) antagonist. When AR binds to enzalutamide, it cannot be normally transported into the cell nucleus or recruit coactivators, resulting in reduced AR transcriptional activity. Enzalutamide has a stronger affinity for AR as compared to the first-generation AR antagonist bicalutamide. Although the approval of drugs such as abiraterone acetate and enzalutamide is of great significance for the treatment of mCRPC, most patients ultimately develop drug resistance after a period of treatment.

Due to the issues of tolerability and safety of therapeutic medicaments, the treatment of prostate cancer remains a clinical challenge, and exploring effective treatment methods is urgently needed.

### SUMMARY

The compound of general formula (I) of the present invention is a kinase inhibitor, and through researches, it has been found that the compound of general formula (I) has a therapeutic effect on prostate cancer, and further has a significant therapeutic effect on castration resistant prostate cancer and castration sensitive prostate cancer as well as metastatic castration resistant prostate cancer.

In order to achieve the above objective, the present invention provides the following solutions:
The present invention provides use of a compound of general formula (I) or a pharmaceutically acceptable salt, stereoisomer or crystal form thereof in the manufacture of a medicament for treating and/or preventing prostate cancer: 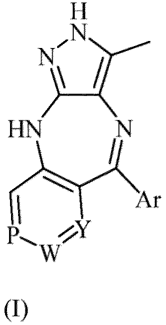 wherein Ar is phenyl optionally substituted with 1 to 3 R₆, and each R₆ is independently selected from hydrogen, amino, cyano, halogen, C₁₋₄ alkyl, and trifluoromethyl;
Y is CR₃;
P is CR₄;
W is N;
R₃ is hydrogen or C₁₋₄ alkyl;
R₄ is -(CH₂)ₙ-(5-11) membered heterocyclyl, wherein n = 0-6, a ring-forming S atom in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

In some embodiments, Ar is phenyl optionally substituted with 1 to 3 R₆, and each R₆ is independently selected from hydrogen and halogen;
Y is CR₃;
P is CR₄;
W is N;
R₃ is hydrogen;
R₄ is selected from -(CH₂)ₙ-(5-6) membered monocyclic heterocyclyl and - (CH₂)ₙ-(7-11) membered polycyclic heterocyclyl, wherein n = 0-6, a ring-forming S atom in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

In a further embodiment, the (5-6) membered monocyclic heterocyclyl is (5-6) membered saturated monocyclic heterocyclyl, and the (7-11) membered polycyclic heterocyclyl is (7-11) membered saturated polycyclic heterocyclyl. In a preferred embodiment, the (7-11) membered saturated polycyclic heterocyclyl is (7-11) membered saturated ortho-fused heterocyclyl, (7-11) membered saturated spiro-heterocyclyl, or (7-11) membered saturated bridged heterocyclyl.

In some embodiments, R⁴ is 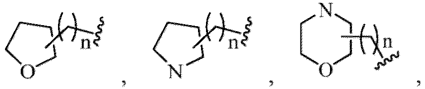 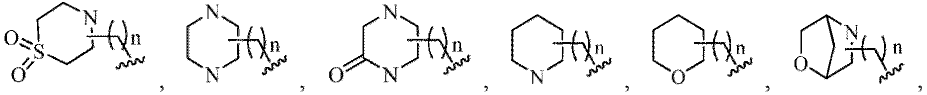 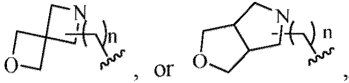 and n = 0-3, wherein the heterocyclyl is optionally substituted with one or more substituents independently selected from C₁₋₃ alkyl and C₃₋₆ cycloalkyl;
in a preferred embodiment, R⁴ is preferably 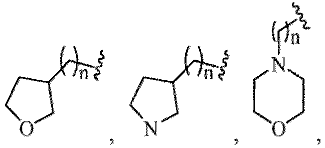 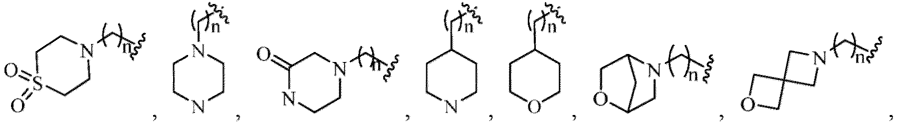 or 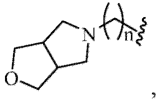 and n = 0-3, wherein the heterocyclyl is optionally substituted with one or more substituents independently selected from C₁₋₃ alkyl and C₃₋₆ cycloalkyl.

In one embodiment, the compound of general formula (I) is a compound shown in Table 1 or a pharmaceutically acceptable salt, stereoisomer or crystal form thereof.

**Table 1. Compounds of the present invention**

| No. | Structure | No. | Structure |
|---|---|---|---|
| 22 | 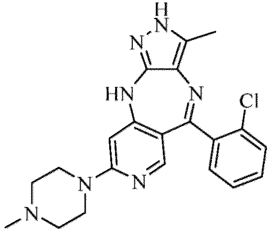 | 29 | 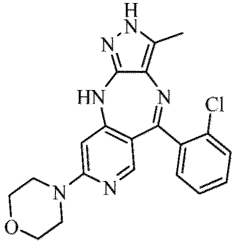 |
| 33 | 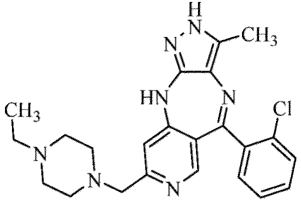 | 34 | 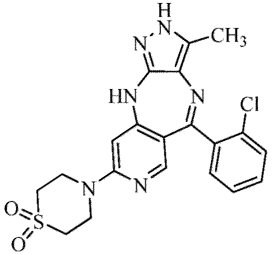 |
| 35 | 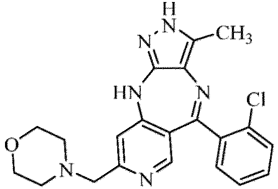 | 36 | 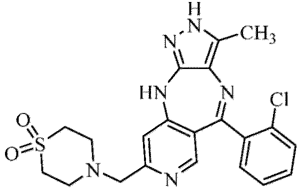 |
| 37 | 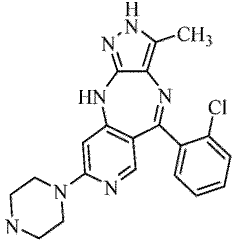 | 38 | 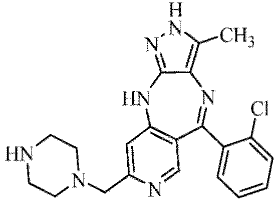 |
| 39 | 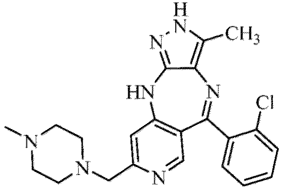 | 40 | 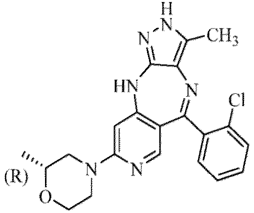 |
| 41 | 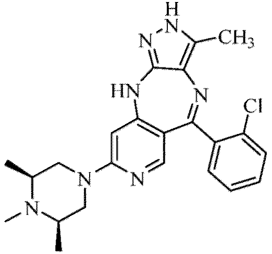 | 42 | 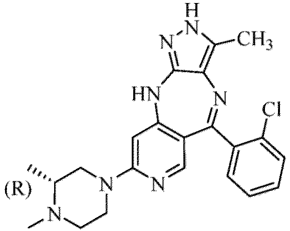 |
| 43 | 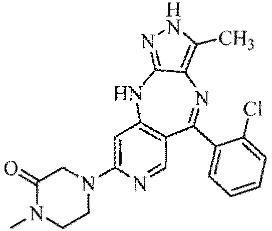 | 44 | 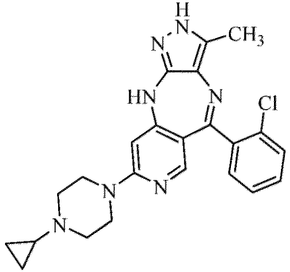 |
| 45 | 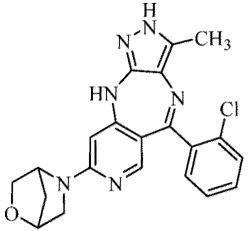 | 46 | 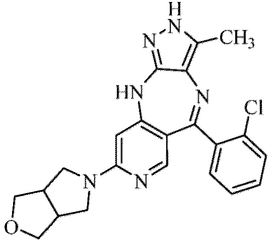 |
| 47 | 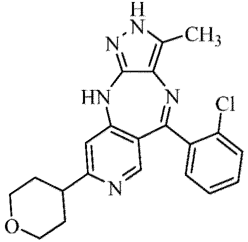 | 48 | 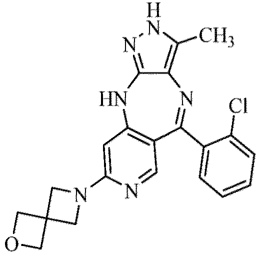 |
| 49 | 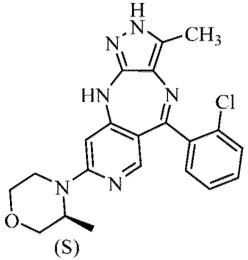 | 50 | 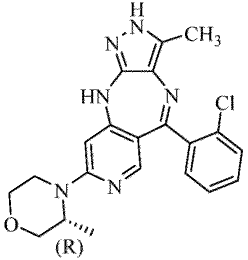 |
| 51 | 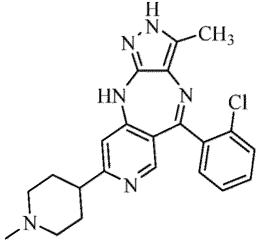 | | |

In some embodiments, the compound of the present invention may also include compounds other than the compound of general formula (I), for example, the specific compounds shown in the table below.

| | | | |
|---|---|---|---|
| 1 | 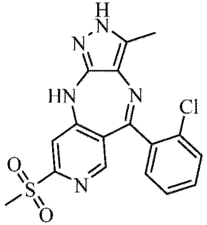 | 2 | 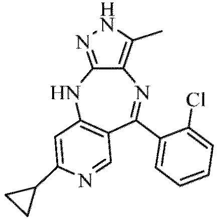 |
| 3 | 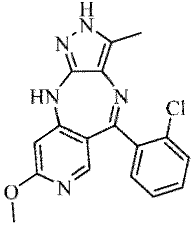 | 4 | 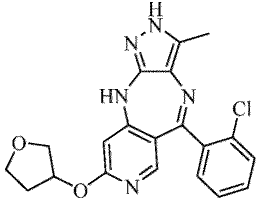 |
| 5 | 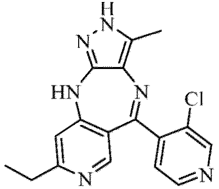 | 6 | 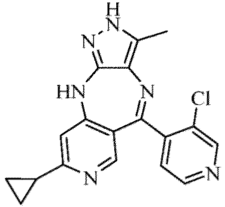 |
| 7 | 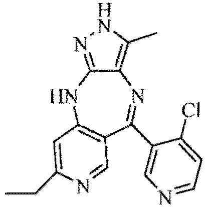 | 8 | 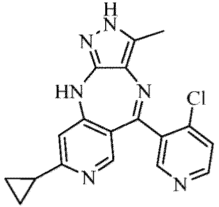 |

| | | | |
|---|---|---|---|
| 9 | 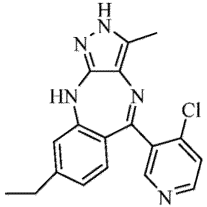 | 10 | 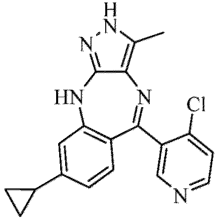 |
| 11 | 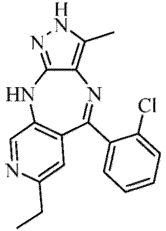 | 12 | 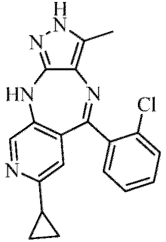 |
| 13 | 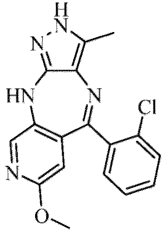 | 14 | 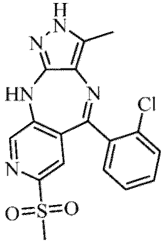 |
| 15 | 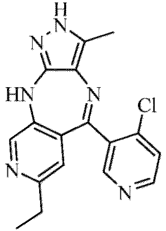 | 16 | 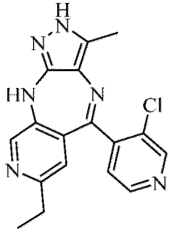 |
| 17 | 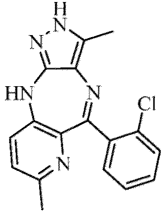 | 18 | 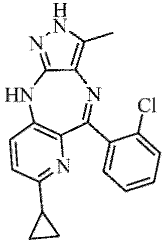 |

| | | | |
|---|---|---|---|
| 19 | 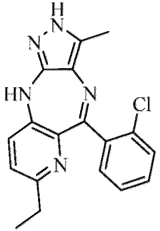 | 20 | 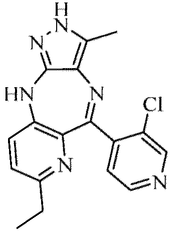 |
| 21 | 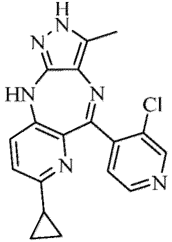 | 30 | 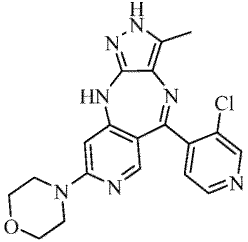 |
| 23 | 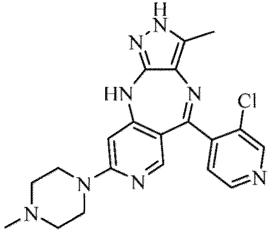 | 24 | 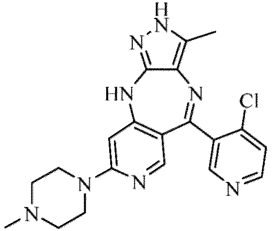 |
| 25 | 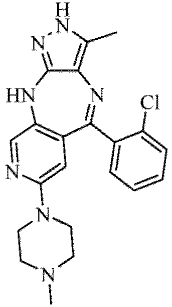 | 26 | 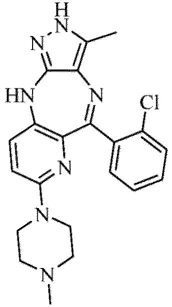 |
| 27 | 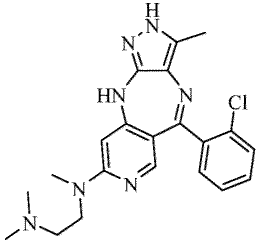 | 28 | 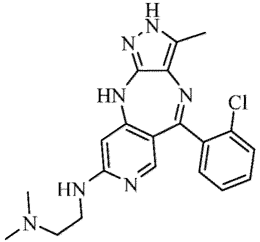 |

| | | | |
|---|---|---|---|
| 31 | 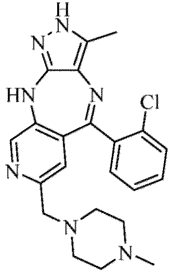 | 32 | 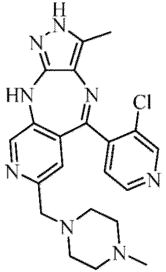 |

In some embodiments, the compound is 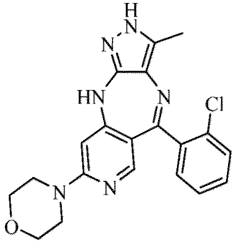 or a pharmaceutically acceptable salt, stereoisomer or crystal form thereof.

The present invention further provides a pharmaceutical composition or pharmaceutical combination product comprising a therapeutically effective amount of a compound of general formula (I) or a pharmaceutically acceptable salt, stereoisomer or crystal form thereof, and one or more pharmaceutically acceptable carriers, diluents or excipients. Optionally, the pharmaceutical composition or pharmaceutical combination product further comprises one or more additional active agents. The definition of the compound of general formula (I) is as described above.

The present invention further provides a method for treating prostate cancer, comprising administering to a prostate cancer patient in need a therapeutically effective amount of a compound of general formula (I) or a pharmaceutically acceptable salt, stereoisomer or crystal form thereof, or the pharmaceutical composition or pharmaceutical combination product described herein, wherein the definition of the compound of general formula (I) is as described above.

The present invention further provides use of a compound of general formula (I) or a pharmaceutically acceptable salt, stereoisomer or crystal form thereof, or the pharmaceutical composition or pharmaceutical combination product described herein in preventing and/or treating prostate cancer, wherein the definition of the compound of general formula (I) is as described above.

The present invention further provides use of a compound of general formula (I) or a pharmaceutically acceptable salt, stereoisomer or crystal form thereof, or the pharmaceutical composition or pharmaceutical combination product described herein in the manufacture of a medicament for preventing and/or treating prostate cancer, wherein the definition of the compound of general formula (I) is as described above.

The present invention further provides a compound of general formula (I) or a pharmaceutically acceptable salt, stereoisomer or crystal form thereof, or the pharmaceutical composition or pharmaceutical combination product described herein for use in the prevention and/or treatment of prostate cancer, wherein the definition of the compound of general formula (I) is as described above.

In some embodiments, the prostate cancer is metastatic prostate cancer.

In some embodiments, the prostate cancer is non-metastatic prostate cancer.

In some embodiments, the prostate cancer is early-stage prostate cancer.

In some embodiments, the prostate cancer is advanced prostate cancer.

In some embodiments, the prostate cancer is one that has not received standard therapy.

In some embodiments, the prostate cancer is one that has failed standard therapy.

In some embodiments, the prostate cancer is one that has failed chemotherapy.

In some embodiments, the prostate cancer is one that has failed endocrine therapy.

In some embodiments, the prostate cancer is one that has failed androgen deprivation therapy.

In some embodiments, the prostate cancer is one that has failed treatment with an androgen synthesis inhibitor and/or an androgen receptor inhibitor.

In some embodiments, the prostate cancer is one that has failed treatment with abiraterone.

In some embodiments, the prostate cancer is one that has failed treatment with enzalutamide, apalutamide, darolutamide, and/or bicalutamide.

In some embodiments, the prostate cancer is early-stage and/or non-metastatic prostate cancer.

In some embodiments, the early-stage and/or non-metastatic prostate cancer is one that has not received standard therapy.

In some embodiments, the prostate cancer is advanced and/or metastatic prostate cancer.

In some embodiments, the advanced and/or metastatic prostate cancer is one that has not received standard therapy.

In some embodiments, the advanced and/or metastatic prostate cancer is one that has failed standard therapy.

In some embodiments, the prostate cancer is castration sensitive prostate cancer.

In some embodiments, the prostate cancer is castration resistant prostate cancer.

In some embodiments, the castration sensitive prostate cancer or the castration resistant prostate cancer is one that has not received standard therapy.

In some embodiments, the castration sensitive prostate cancer or the castration resistant prostate cancer is one that has failed standard therapy.

In some embodiments, the castration sensitive prostate cancer or the castration resistant prostate cancer is one that has failed chemotherapy.

In some embodiments, the castration sensitive prostate cancer or the castration resistant prostate cancer is one that has failed endocrine therapy.

In some embodiments, the castration sensitive prostate cancer or the castration resistant prostate cancer is one that has failed treatment with androgen deprivation therapy.

In some embodiments, the castration sensitive prostate cancer or the castration resistant prostate cancer is one that has failed treatment with an androgen synthesis inhibitor and/or an androgen receptor inhibitor.

In some embodiments, the castration sensitive prostate cancer or the castration resistant prostate cancer is one that has failed treatment with abiraterone.

In some embodiments, the castration sensitive prostate cancer or the castration resistant prostate cancer is one that has failed treatment with enzalutamide, apalutamide, darolutamide, and/or bicalutamide.

In some embodiments, the prostate cancer is metastatic castration sensitive prostate cancer.

In some embodiments, the prostate cancer is non-metastatic castration sensitive prostate cancer.

In some embodiments, the non-metastatic or metastatic castration sensitive prostate cancer is one that has not received standard therapy.

In some embodiments, the non-metastatic or metastatic castration sensitive prostate cancer is one that has failed standard therapy.

In some embodiments, the prostate cancer is metastatic castration resistant prostate cancer.

In some embodiments, the prostate cancer is a non-metastatic castration resistant prostate cancer.

In some embodiments, the non-metastatic or metastatic castration resistant prostate cancer is one that has not received standard therapy.

In some embodiments, the non-metastatic or metastatic castration resistant prostate cancer is one that has failed standard therapy.

In some embodiments, the non-metastatic or metastatic castration resistant prostate cancer is one that has failed chemotherapy.

In some embodiments, the non-metastatic or metastatic castration resistant prostate cancer is one that has failed endocrine therapy.

In some embodiments, the non-metastatic or metastatic castration resistant prostate cancer is one that has failed androgen deprivation therapy.

In some embodiments, the non-metastatic or metastatic castration resistant prostate cancer is one that has failed treatment with an androgen synthesis inhibitor and/or an androgen receptor inhibitor.

In some embodiments, the non-metastatic or metastatic castration resistant prostate cancer is one that has failed treatment with abiraterone.

In some embodiments, the non-metastatic or metastatic castration resistant prostate cancer is one that has failed treatment with enzalutamide, apalutamide, darolutamide, and/or bicalutamide.

In some embodiments, the metastatic castration prostate cancer is histopathologically or cytologically confirmed prostate cancer that has previously failed initial continuous androgen deprivation therapy and that has been confirmed by imaging to have a distinct metastatic focus in bone or soft tissue.

In some embodiments, the metastatic castration prostate cancer is histopathologically or cytologically confirmed metastatic castration resistant prostate cancer for which no standard therapy is available.

In some embodiments, the present invention further provides a dose and a dosing regimen for the treatment of a prostate cancer patient with the compound of general formula (I) or the pharmaceutically acceptable salt, stereoisomer or crystal form thereof, in particular as follows:

In some embodiments, the compound of general formula (I) or the pharmaceutically acceptable salt, stereoisomer or crystal form thereof is administered at a single dose of 3 mg to 20 mg, such as 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, and is administered once daily, twice daily, once every two days, or once every three days, preferably once daily.

In some embodiments, the compound of general formula (I) or the pharmaceutically acceptable salt, stereoisomer or crystal form thereof may be further prepared with one or more pharmaceutically acceptable carriers into any pharmaceutically acceptable pharmaceutical formulation.

In some embodiments, the pharmaceutical formulation may comprise one or more pharmaceutically acceptable carriers, and may be administered to a patient or subject in need of such treatment by oral, parenteral, rectal, or transpulmonary administration, and the like. For oral administration, the pharmaceutical composition may be prepared into a conventional solid formulation, such as a tablet, a capsule, a pill, and a granule; or may also be prepared into an oral liquid formulation, such as an oral solution, an oral suspension, and a syrup. In the preparation of an oral formulation, an appropriate filler, binder, disintegrant, lubricant, and the like may be added. For parenteral administration, the pharmaceutical composition may be prepared into an injection, including a solution injection, a sterile powder for injection, and a concentrated solution for injection. The injection can be produced by a conventional method existing in the pharmaceutical field, and during the preparation process, no additive may be added, or an appropriate additive may be added according to the properties of the medicament. For rectal administration, the pharmaceutical composition may be prepared into a suppository or the like. For transpulmonary administration, the pharmaceutical composition may be prepared into an inhalant, a spray, or the like.

"Treat, treating, or treatment" described herein refers to administering to a patient an effective amount of a medicament that slows or cures an undesired physiological change or condition in the patient, such as a hyperproliferative condition, such as the growth, formation, or spread of a cancer, to achieve a favorable or desired clinical result, including, but not limited to: alleviation of symptoms, diminishment of the disease, stabilization (i.e., not worsening) of the disease state, delay or slowing of the disease progression, amelioration or palliation of the disease state, and regression (whether partial or complete) of the disease, whether detectable or undetectable.

"Treat, treating, or treatment" described herein may include neoadjuvant treatment and adjuvant treatment.

"Neoadjuvant treatment" refers to systemic treatment for a patient with an original tumor prior to planned surgery or local treatment with surgery plus radiotherapy, and treatment means of the neoadjuvant treatment may be chemotherapy, endocrine, targeting, immunization, and radiotherapy depending on the tumor types. The purpose of neoadjuvant treatment is to provide immediate systemic treatment, thereby potentially eradicating micrometastases that otherwise proliferate when following the standard sequence of surgery followed by systemic treatment. The neoadjuvant treatment may also help reduce tumor size, allowing complete resection of an initially unresectable tumor or preservation of portions of the organ and its functions. In addition, the neoadjuvant treatment allows for *in-vivo* evaluation of the efficacy of a medicament, which may guide the choice of subsequent treatments.

"Adjuvant treatment" refers to a treatment given after definitive surgery (where evidences of residual diseases cannot be detected) to destroy any residual cancer cells in a body, for use in the reduction of the likelihood of tumor relapse or dissemination to other sites. Treatment means of the adjuvant treatment are about the same as those of the neoadjuvant treatment. The objective of the adjuvant treatment is to prevent cancer relapse and thus reduce the chance of cancer-related death. The adjuvant treatment specifically excludes the neoadjuvant treatment herein.

"Failed", "failed treatment" or "failed chemotherapy" described herein refers to the occurrence of progressive disease (progressive disease (PD) assessed according to the RECIST 1.1 response evaluation criteria in tumor tissues or progressive disease (PD) assessed according to the prostate cancer bone lesion evaluation criteria PCWG3) during the treatment or after the last treatment, or intolerance during the treatment due to toxic side effects.

The "intolerance due to toxic side effects" described herein refers to the inability to continue the treatment due to adverse reactions caused by the medicament.

The "standard therapy" described herein includes androgen deprivation therapy (surgical castration as well as medical castration treatments including, but not limited to, goserelin, leuprorelin, triptorelin, degarelix, etc.), chemotherapy, systemic radiotherapy, and novel endocrine therapy.

The "chemotherapy" described herein includes, but is not limited to, docetaxel, the combination of docetaxel and prednisone, and the like.

The "treatment with abiraterone" described herein includes, but is not limited to, abiraterone, the combination of abiraterone and prednisone, and the like.

The "endocrine therapy" described herein is a method for treating prostate cancer by inhibiting or reducing androgen (receptor) activity, which includes both traditional androgen deprivation therapy (ADT) and novel endocrine therapy. ADT includes surgical castration treatments (such as orchiectomy) and medical castration treatments (including, but not limited to, the administration of goserelin, leuprorelin, triptorelin, degarelix, etc.). Novel endocrine therapy includes androgen synthesis inhibitors (such as abiraterone) and androgen receptor inhibitors (such as enzalutamide, apalutamide, darolutamide, bicalutamide, etc.).

Progression-free survival (PFS): in the efficacy analysis set, the time from the first use of the study medicament to the occurrence of disease progression or death due to any cause.

Overall survival (OS): the time from the start of treatment with the study medicament to the occurrence of death due to any cause. For patients who have not died at the time of analysis, the date on which their survival was last known will be used for review.

Objective response rate (ORR): the proportion of patients whose best response is CR or PR at the time of discontinuation of the test medicament in all patients.

Duration of response (DOR): the time from the first PR or CR to the first PD or death.

Disease control rate (DCR): the proportion of patients whose best response is CR, PR, or SD.

According to the RECIST1.1 criteria, the response evaluation criteria are divided into complete response (CR), partial response (PR), stable disease (SD), and progressive disease (PD).

Complete response (CR): the disappearance of all target lesions (all pathological lymph nodes must reduce to <10 mm in short axis), the disappearance of non-target lesions, and no appearance of new lesions.

Partial response (PR): a decrease of at least 30% in the sum of diameters of target lesions compared to the baseline level, the disappearance or stabilization of non-target lesions, and no appearance of new lesions.

Progressive disease (PD): a relative increase of at least 20% in the sum of diameters of target lesions, or worsening of non-target lesions, or the appearance of new lesions (the occurrence of any one of the three is considered PD).

Stable disease (SD): increases/decreases in target lesions between PR and PD, the disappearance or stabilization of non-target lesions, and the absence of new lesions.

The "therapeutically effective amount" described herein refers to an amount of the aforementioned compound or the pharmaceutically acceptable salt, stereoisomer or crystal form thereof that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health status of the patient, and the route of administration. For example, a single bolus can be administered, several separate doses can be administered over time, or the dose can be proportionally reduced or increased as indicated by the urgent need for treatment. It should be noted that the dose values may vary depending on the type and severity of the condition to be alleviated, and can include single or multiple doses. For any particular individual, the specific dosing regimen will be adjusted over time according to the individual's needs and the professional judgment of a person administering a composition or supervising the administration of a composition.

The "subject" or "individual" in the prevention and/or treatment of prostate cancer refers to any animal classified as a mammal, including human, domestic and farm animals, and zoo, sports or pet animals, such as dogs, horses, cats, and cattle. Preferably, the mammal is a human. The subject or individual may be a patient.

### Definition

The "halogen" described herein refers to fluorine, chlorine, bromine, iodine, and the like, and preferably fluorine and chlorine.

The "halogenated" described herein means that any hydrogen atom in a substituent can be substituted with one or more identical or different halogen atoms. The "halogen" is as defined above.

The "cyano" described herein refers to the -CN group.

The "amino" described herein refers to the -NH₂ group.

The "C₁₋₄ alkyl" described herein refers to linear or branched alkyl derived from a hydrocarbon moiety having 1 to 4 carbon atoms by removing one hydrogen atom, such as methyl, ethyl, n-propyl, isopropyl, n-butyl, isobutyl, sec-butyl, or tert-butyl. The "C₁₋₃ alkyl" refers to the above alkyl having 1 to 3 carbon atoms.

The "C₃₋₆ cycloalkyl" described herein refers to a monocyclic cycloalkyl or bicyclic cycloalkyl system or a polycyclic cycloalkyl system having 3 to 6 carbon atoms. These groups are saturated but not aromatic, including monocyclic and polycyclic ring structures which can be formed, unless otherwise specified; examples thereof include, but are not limited to: cyclopropyl, cyclobutyl, cyclopentyl, cyclohexyl, bicyclo[2.2.2]hexane, and bicyclo[3.2.1]hexane.

The "5-11 membered heterocyclyl" described herein refers to a non-aromatic cyclic group having 5 to 11 ring carbon atoms, wherein at least one ring carbon atom is substituted with one or more heteroatoms selected from O, S and N, and preferably 1 to 3 heteroatoms, and ring-forming atoms including carbon atoms, nitrogen atoms and sulfur atoms may be oxidized.

The "heterocyclyl" refers to a monocyclic heterocyclyl or bicyclic heterocyclyl system or a polycyclic heterocyclyl system, including saturated and partially saturated heterocyclyl, but not including aromatic rings. Unless otherwise specified, the "5-11 membered heterocyclyl" described herein includes monocyclic and polycyclic ring structures which can be formed.

The monocyclic heterocyclyl may be 5-7 membered heterocyclyl, 5-6 membered heterocyclyl, 5-6 membered oxygen-containing heterocyclyl, 5-6 membered nitrogen-containing heterocyclyl, 5-6 membered saturated heterocyclyl, etc. Examples of the 5-6 membered monocyclic heterocyclyl described herein include, but are not limited to, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H-*imidazolyl, 4,5-dihydro-1*H*-imidazolyl, 4,5-dihydro-1*H*-pyrazolyl, 4,5-dihydro-3*H-*pyrazolyl, 4,5-dihydrothiazolyl, 2,5-dihydrothiazolyl, 2*H*-pyranyl, 4*H*-pyranyl, 2*H-*thiopyranyl, 4*H*-thiopyranyl, 2,3,4,5-tetrahydropyridyl, 1,2-isoxazolyl, 1,4-isoxazolyl, 6*H-*1,3-oxazinyl, etc.

The polycyclic heterocyclyl includes ortho-fused heterocyclyl, spiro-heterocyclyl, and bridged heterocyclyl, which may be saturated, partially saturated, or unsaturated, but non-aromatic. Unless otherwise specified, the 7-11 membered polycyclic heterocyclyl described herein includes ortho-fused, spiro-, and bridged structures which can be formed.

The ortho-fused heterocyclyl may be 7-11 membered ortho-fused cyclyl, preferably 7-11 membered saturated ortho-fused cyclyl, and examples thereof include, but are not limited to: 3,6-diazabicyclo[3.2.0]heptyl, 3,8-diazabicyclo[4.2.0]octyl, 3,7-diazabicyclo[4.2.0]octyl, octahydropyrrolo[3,4-*c*]pyrrolyl, octahydropyrrolo[3,4-*b*]pyrrolyl, octahydropyrrolo[3,4-*b*][1,4]oxazinyl, octahydro-1*H*-pyrrolo[3,4-*c*]pyridyl, 2,3-dihydrobenzofuran-2-yl, 2,3-dihydrobenzofuran-3-yl, indolin-1-yl, indolin-2-yl, indolin 3-yl, 2,3-dihydrobenzothien-2-yl, octahydro-1*H*-indolyl, and octahydrobenzofuranyl.

The spiro-heterocyclyl may be 7-11 membered spiro-heterocyclyl, preferably 7-11 membered saturated spiro-heterocyclyl, and examples thereof include, but are not limited to:

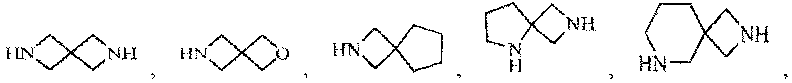

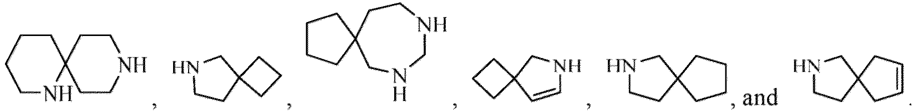

The bridged heterocyclyl may be 7-11 membered bridged heterocyclyl, preferably 7-11 membered saturated bridged heterocyclyl, and examples thereof include, but are not limited to:

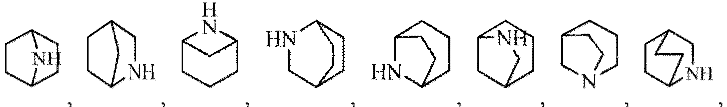

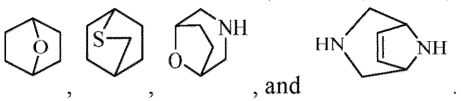

The "pharmaceutically acceptable salt" described herein refers to a pharmaceutically acceptable addition salt of acid and base and a solvate. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH₂)n-COOH (wherein n = 0-4)), etc. Such pharmaceutically acceptable salts further include salts of the following bases: sodium, potassium, calcium, ammonium, etc. Those skilled in the art know a variety of pharmaceutically acceptable non-toxic addition salts.

All numerical ranges described herein include both endpoints of the ranges, all integers within the range, and subranges formed by these integers. For example, "5-11 membered" includes 5, 6, 7, 8, 9, 10 or 11 membered, "5-6 membered" includes 5 or 6 membered, and "7-11 membered" includes 7, 8, 9, 10 or 11 membered and so on.

The "one or more" as described herein with respect to a substituent refers to the number of substituents with which all positions can be chemically substituted in the substituted group, preferably 1 to 6, more preferably 1 to 5, more preferably 1 to 3, and more preferably 1 to 2.

The "crystal form" described herein may be prepared from the compound of general formula (I) by conventional methods for preparing crystal forms used in the art.

The "stereoisomer" of the compound of general formula (I) described herein means that an enantiomer can be formed when asymmetric carbon atoms are present in the compound of general formula (I); a cis-trans isomer can be formed when a carbon-carbon double bond or a ring structure is present in the compound; a tautomer can be formed when a ketone or oxime is present in the compound. All enantiomers, diastereomers, racemates, cis-trans isomers, tautomers, geometric isomers, and epimers of the compound of general formula (I) as well as mixtures thereof are included in the scope of the present invention.

The preparation of the compound of general formula (I) described herein can be found in the detailed description of WO2018108079A1.

### BENEFICIAL EFFECTS OF PRESENT INVENTION

The compound of general formula (I) or the pharmaceutically acceptable salt, stereoisomer or crystal form thereof of the present invention has a therapeutic effect on prostate cancer; further, it has a significant therapeutic effect on castration resistant prostate cancer and castration sensitive prostate cancer; even further, it also has a significant therapeutic effect on metastatic castration resistant prostate cancer, demonstrating good clinical application potential.

In order to make the objective, technical schemes, and advantages of the present invention more apparent, the present invention is further described in detail below. Obviously, the embodiments described herein are only some, but not all, of embodiments of the present invention. Based on the embodiments of the present invention, all other embodiments obtained by those of ordinary skill in the art without creative work shall fall within the protection scope of the present invention.

The abbreviations and English expressions used in the present invention have the following meanings:

| Abbreviation/English | Meaning |
|---|---|
| DMSO | Dimethyl sulfoxide |
| MC | Methyl cellulose |
| Qd | Once-daily administration |
| mCRPC | Metastatic castration resistant prostate cancer |

### Experimental Example 1. Assay on Cell Activity of Compound of the Present Invention

Test sample: compound 29 of the present invention, the structure of which is shown above, and which was prepared as described in the detailed description of WO2018108079A1.
Prostate cancer cell materials: DU145, PC-3, VCaP, and LNCaP clone FGC.
Test instrument: PE Envision multimode microplate reader.

### Test method:

Each cell line was seeded in a 384-well plate for adherent culture overnight, and then compounds at different concentrations (10 dose groups, serial 4-fold dilution with DMSO) were added to give a maximum final concentration of 10000 nM, wherein the final DMSO content was 0.33%, and staurosporine at a final concentration of 10 µM was used as a system positive control (100% inhibition). Negative control wells contained medium with the same amount of DMSO (0% inhibition). The plate was incubated at 37 °C, 5% CO₂ and 95% humidity for 72 h for later test. 30 µL of Cell titer-Glo reagent was added to each well, and after the plate was incubated at room temperature for 30 min, the final data were read using the microplate reader.

The data were calculated as follows: Inhibition rate (%) = 100% - (compound signal - system positive control signal)/(negative control signal - system positive control signal) × 100%.

The test results are shown in Table 2, wherein B: 0-1000 nM; C: 1000-10000 nM; D: >10000 nM.

**Table 2. Inhibitory activity of compound 29 of the present invention against prostate cancer cell proliferation**

| Cell | Compound IC₅₀/nM |
|---|---|
| DU145 | B |
| PC-3 | B |
| VCaP | B |
| LNCaP clone FGC | C |

It can be seen from the experimental results in Table 2 that compound 29 of the present invention had good anti-tumor proliferation activity against prostate cancer cell lines, demonstrating good potential for clinical treatment of prostate cancer.

### Experimental example 2. In-Vivo Pharmacodynamic Study of Compound of the Present Invention on Human Castration Resistant Prostate Cancer PDX Subcutaneous Tumor Model

Test sample: compound 29 of the present invention, the structure of which is shown above, and which was prepared as described in the detailed description of WO2018108079A1. Abiraterone was obtained from commercially available sources.

Animals, information on the patient from whom the tumor masses were derived, and other materials: human prostate cancer tumor sample LD1-0034-361929, derived from a male patient resistant to abiraterone; clinical diagnosis: castration resistant prostate cancer with bladder metastatic lesions; pathological diagnosis: prostate cancer-poorly differentiated adenocarcinoma;
male Nu/Nu nude mice, aged 6-8 weeks.

### Test method:

### 1. Construction and grouping of tumor-bearing mice

The tumor masses were inoculated into the right back of mice, and the animals were weighed before administration. Tumor volumes were measured, and grouping was designed in blocks according to the tumor volumes.

### 2. Dosing regimen

Administration was carried out as in the table below.

| Group | Dose (mg/kg) | Number of animals | Route of administration | Frequency of administration | Administration period |
|---|---|---|---|---|---|
| Vehicle control | 0 | 5 | Oral intragastric administration | Qd * 7 days/week | 39 days |
| Abiraterone | 200 | 5 | Oral intragastric administration | Qd * 7 days/week | 39 days |
| Compound 29 | 15 | 5 | Oral intragastric administration | Qd * 7 days/week | 39 days |

| | | | | | |
|---|---|---|---|---|---|
| Notes: vehicle control: 0.5% MC; formula of compound 29: 0.5% MC; formula of abiraterone: 10% DMSO + 90% vegetable oil | | | | | |

### 3. Experimental observation index

The animals were monitored daily for health status and mortality, body weight and tumor volume were measured twice a week, and samples were collected after the last administration. The therapeutic effect on tumor volume was evaluated by TGI%, wherein the relative tumor inhibition rate TGI (%): TGI = 1 - T/C (%). T/C % is the relative tumor proliferation rate, i.e., the percentage of the relative tumor volume in the treatment and control groups at a given time point. T and C are relative tumor volumes (RTVs) of the treatment and control groups at a given time point, respectively. The calculation formula is as follows: T/C % = T_{RTV}/C_{RTV} × 100% (T_{RTV}: mean RTV for treatment group; C_{RTV}: mean RTV for vehicle control group; RTV = Vₜ/V₀, wherein V₀ is the tumor volume of the animal at the time of grouping, and Vₜ is the tumor volume of the animal after treatment).

The results are shown in Table 3 below:

**Table 3. Effect of compound 29 of the present invention on tumor growth in a human prostate cancer LD1-0034-361929 subcutaneous xenograft tumor model**

| Group | Number of animals | Tumor volume (mm³) | TGI (%) |
|---|---|---|---|
| Vehicle control | 5 | 1404.87±483.33 | -- |
| Abiraterone | 5 | 1549.43±533.15 | -8.97 |
| Compound 29 | 5 | 622.23±247.3 | 58.07 |

It can be seen from the experimental results in Table 3 that compound 29 had a significant inhibitory effect on the abiraterone-resistant human castration resistant prostate cancer PDX subcutaneous tumor model LD1-0034-361929, indicating that compound 29 has good clinical application potential for the treatment of abiraterone-resistant mCRPC tumors.

### Experimental example 3. Clinical Study of Compound of the Present Invention in the Treatment of Patients with Metastatic Castration Resistant Prostate Cancer

Test medicament: compound 29, the structure of which is shown above, and which was prepared as described in the detailed description of WO2018108079A1.

Inclusion criteria: patients with histopathologically or cytologically confirmed metastatic castration resistant prostate cancer for which no standard therapy is available.

Dosing regimen: compound 29 was administered orally at a prescribed dose (such as 8 mg, 10 mg, or 12 mg) once daily, with 28 consecutive days of administration being one cycle.

Response evaluation: among the 11 patients with response evaluation data (10 patients received a single dose of 12 mg, and 1 patient received a single dose of 10 mg), 7 patients achieved partial response (PR), and 2 patients reported stable disease (SD).

This study indicates that compound 29 has a very good clinical therapeutic effect on metastatic castration resistant prostate cancer.

The above description is only for the purpose of illustrating preferred examples of the present invention, and is not intended to limit the scope of the present invention. Any modifications, equivalents, improvements, and the like made without departing from the spirit and principle of the present invention should be included in the protection scope of the present invention.

## Claims

1. Use of a compound of general formula (I) or a pharmaceutically acceptable salt, stereoisomer or crystal form thereof in the manufacture of a medicament for treating and/or preventing prostate cancer: 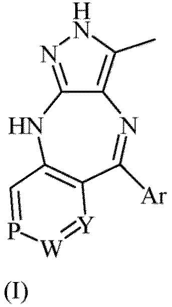 wherein Ar is phenyl optionally substituted with 1 to 3 R₆, and each R₆ is independently selected from hydrogen, amino, cyano, halogen, C₁₋₄ alkyl, and trifluoromethyl;
Y is CR₃;
P is CR₄;
WisN;
R₃ is hydrogen or C₁₋₄ alkyl;
R₄ is -(CH₂)ₙ-(5-11) membered heterocyclyl, wherein n = 0-6, a ring-forming S atom in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

2. The use according to claim 1,
wherein Ar is phenyl optionally substituted with 1 to 3 R₆, and each R₆ is independently selected from hydrogen and halogen;
Y is CR₃;
P is CR₄;
WisN;
R₃ is hydrogen;
R₄ is selected from -(CH₂)ₙ-(5-6) membered monocyclic heterocyclyl and -(CH₂)ₙ-(7-11) membered polycyclic heterocyclyl, wherein n = 0-6, a ring-forming S atom in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

3. The use according to claim 2,
wherein
R₄ is 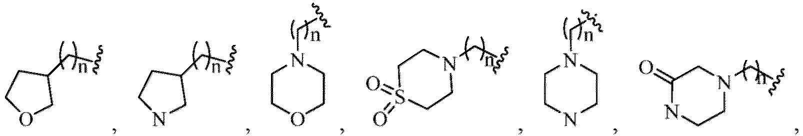 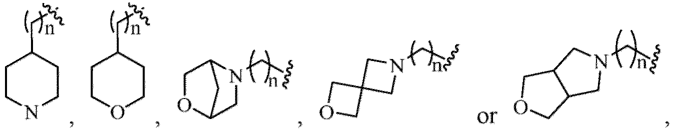 and n = 0-3, wherein the heterocyclyl is optionally substituted with one or more substituents independently selected from C₁₋₃ alkyl and C₃₋₆ cycloalkyl.

4. The use according to claim 3, wherein the compound is selected from compounds of the following structures, and pharmaceutically acceptable salts, stereoisomers and crystal forms thereof:

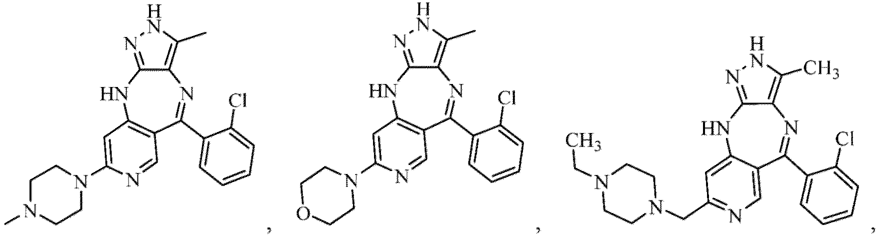

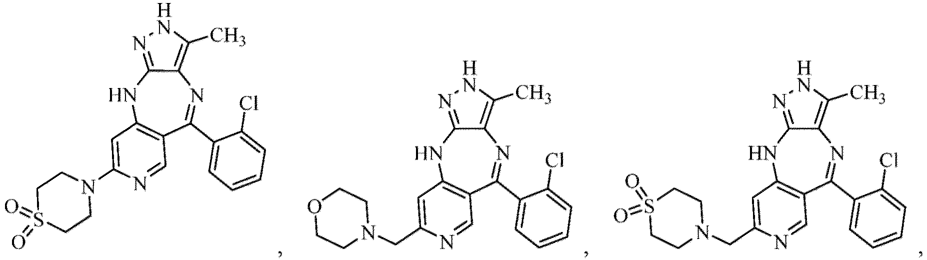

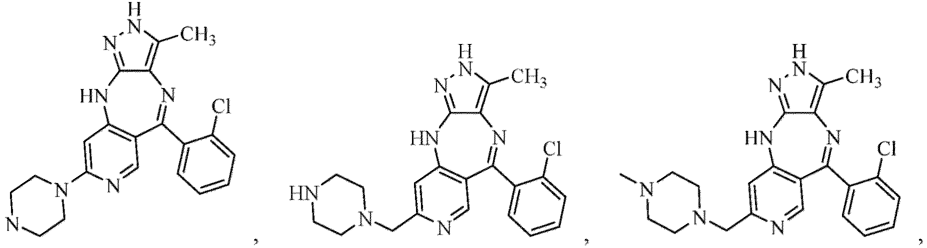

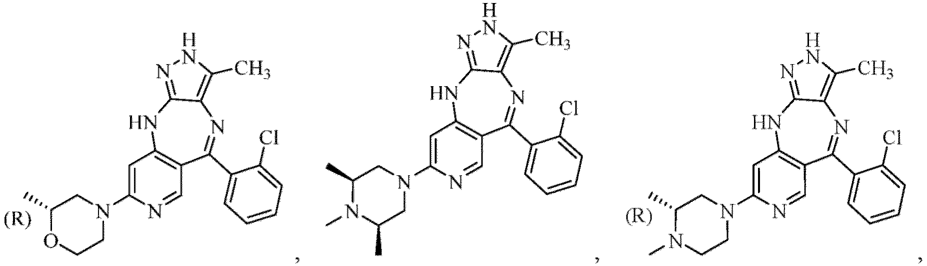

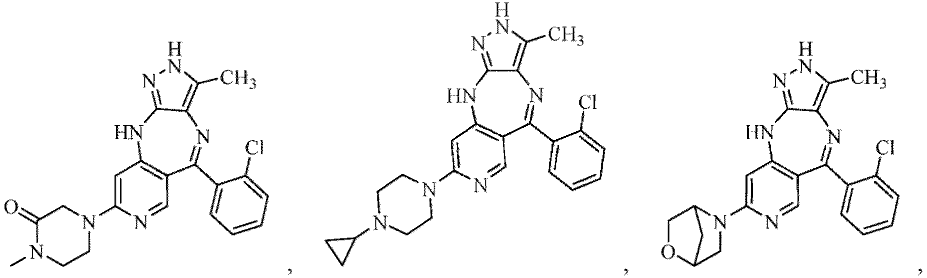

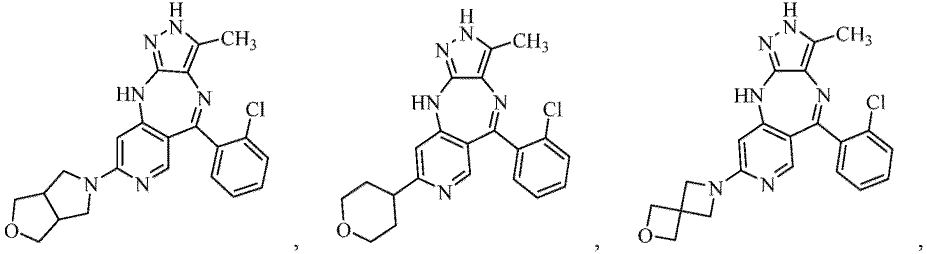

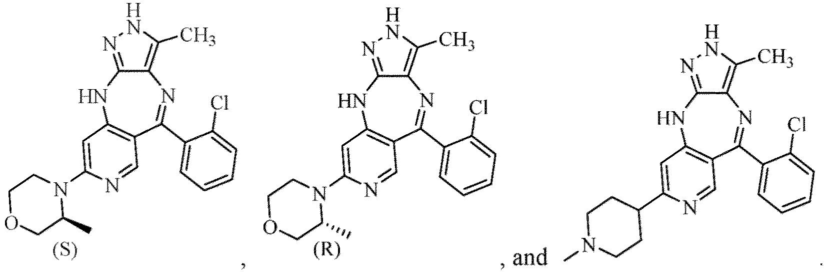

5. The use according to claim 4, wherein the compound is 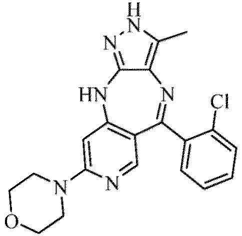 or a pharmaceutically acceptable salt, stereoisomer or crystal form thereof.

6. Use of a composition or combination product comprising the compound or the pharmaceutically acceptable salt, stereoisomer or crystal form thereof according to any one of claims 1 to 5 in the manufacture of a medicament for treating and/or preventing prostate cancer.

7. The use according to any one of claims 1 to 6, wherein the prostate cancer is early-stage or advanced prostate cancer.

8. The use according to any one of claims 1 to 6, wherein the prostate cancer is metastatic or non-metastatic prostate cancer.

9. The use according to any one of claims 1 to 6, wherein the prostate cancer is castration sensitive prostate cancer or castration resistant prostate cancer.

10. The use according to any one of claims 1 to 6, wherein the prostate cancer is non-metastatic castration resistant prostate cancer or metastatic castration resistant prostate cancer.

11. The use according to any one of claims 1 to 6, wherein the prostate cancer is prostate cancer that has failed standard therapy or that has not received standard therapy.

12. Use of compound 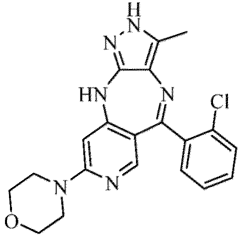 or a pharmaceutically acceptable salt, stereoisomer or crystal form thereof in the manufacture of a medicament for treating and/or preventing metastatic castration resistant prostate cancer.
